# EUROPÄISCHE PATENTSCHRIFT

(11) **EP 0 971 759 B1**
(45) Veröffentlichungstag und Bekanntmachung des Hinweises auf die Patenterteilung: **11.06.2003**
(21) Anmeldenummer: 97904972.3
(22) Anmeldetag: 10.03.1997
(51) Int. Cl.: A61M 5/19, B05C 17/005

(54) **GERÄT ZUR ABGABE VON FLÜSSIGKEITEN**
DEVICE FOR DISPENSING FLUIDS
DISPOSITIF POUR DISTRIBUER DES FLUIDES

(43) Veröffentlichungstag der Anmeldung: 19.01.2000
(73) Patentinhaber: Disetronic Licensing AG, 3401 Burgdorf (CH)
(72) Erfinder: JEANBOURQUIN, Edgar, CH-4623 Neuendorf (CH)
(86) Internationale Anmeldenummer: CH9700092
(87) Internationale Veröffentlichungsnummer: WO98040115

(56) Entgegenhaltungen:
- EP-A- 0 045 339
- WO-A-85/05275
- WO-A-93/06940
- US-A- 4 214 584
- US-A- 5 137 181

## Beschreibung

Die Erfindung bezieht sich auf ein Gerät zur Abgabe von Flüssigkeiten gemäss Oberbegriff des Anspruchs 1.

Geräte zur automatischen Abgabe von Flüssigkeiten sind seit längerem bekannt. Auf eine sich in einem Behälter befindende Flüssigkeit mit meist hoher Viskosität, wie Pasten, Öle, Arzneimittel und dergleichen wirkt eine Kraft derart, dass das Material aus dem Behälter gepresst wird.
Die Kraftquelle ist dabei im Grunde irrelevant, meist wird als Kraftquelle eine Feder oder ein Gas benutzt.
Aus der Patentschrift EP-B-0 435 512 ist ein Abgabegerät für ein Arzneimittel bekannt, in dem eine im Inneren eines Behälters angebrachte Feder direkt auf einen Kolben wirkt, welcher den Federbereich vom Arzneimittelbereich des Gerätes trennt. Durch die Kraft der Feder wird der Kolben in den Arzneimittelbereich geschoben und verdrängt das Arzneimittel. Durch einen Infusionsschlauch wird das Arzneimittel an den gewünschten Ort in den menschlichen Körper transportiert. Der Fluss des Arzneimittels wird durch eine Klemme, welche am Infusionsschlauch angebracht ist, geöffnet oder unterbrochen. Eine andere Art den Arzneimittelfluss zu unterbrechen ist nur mit bedeutend grösserem Aufwand zu realisieren.
Aus den Patentschriften US-A-4 874 368 und WO 93/06940 sind Abgabegeräte bekannt, welche aus zwei parallel angeordneten Behältern Flüssigkeiten abgeben - in den erwähnten Schriften ist ein Behälter mit Fibrin und der andere mit Spritzampullen, in denen ein Arzneimittel zwischen einem beweglichen Kolben und einer Auslassöffnung angebracht ist. Zur Verlängerung der beiden Kolben der parallel angeordneten Spritzampullen dienen zwei mit den Kolben verbundenen Kolbenstangen, welche von einem, die beiden Kolbenstangen haltenden, Deckel gehalten werden. Durch manuelle Kraft auf den Deckel in Richtung Arzneimittel werden die beiden Kolben gleichmässig in Richtung Arzneimittel verschoben, was zur Verdrängung von Arzneimittel aus den Spritzampullen führt. Auf die beiden Auslassöffnungen der Spritzampullen, durch welche das Arzneimittel verdrängt wird, wird eine Doppelkanüle aufgesetzt, welche die beiden Arzneimittel beim Austreten vermischt.

Nachteilig an den bekannten Geräten ist, dass die Kraft zur dosierten Abgabe von Flüssigkeiten entweder manuell erfolgen muss oder im Falle eines automatischen Ausstosses der Flüssigkeit, die Vorrichtung zur Dosierung ungenügend ist.

Hier will die Erfindung Abhilfe verschaffen. Der Erfindung liegt die Aufgabe zugrunde, ein Abgabegerät für Flüssigkeiten zu entwickeln, welches mittels Federantrieb Flüssigkeit aus mindestens zwei Behältern verdrängt, mit einer einfachen Vorrichtung zur Dosierung des Ausstosses ausgestattet ist und kostengünstig hergestellt werden kann.

Die Erfindung löst die gestellte Aufgabe mit einem Abgabegerät, welches die Merkmale des Anspruchs 1 aufweist.

Die durch die Erfindung erreichten Vorteile sind im wesentlichen darin zu sehen, dass mittels einem kostengünstigen automatischen Abgabegerät, eine Dosierung der Ausschüttung von zu mischenden Flüssigkeiten aus zwei oder mehreren Behältern erfolgen kann.

Ein bevorzugtes Ausführungsbeispiel der Erfindung ist in den Figuren dargestellt.

Es zeigen:
- Fig. 1: Ein erfindungsgemässes Abgabegerätes
- Fig. 2: Eine Oberansicht eines erfindungsgemässen Abgabegerätes, mit aufgesetzter Doppelkanüle

In der nachfolgenden Beschreibung werden die Begriffe proximal und distal in der in der Medizin üblichen Weise gebraucht, also proximal = dem Patienten zugewandt und distal = vom Patienten abgewandt.

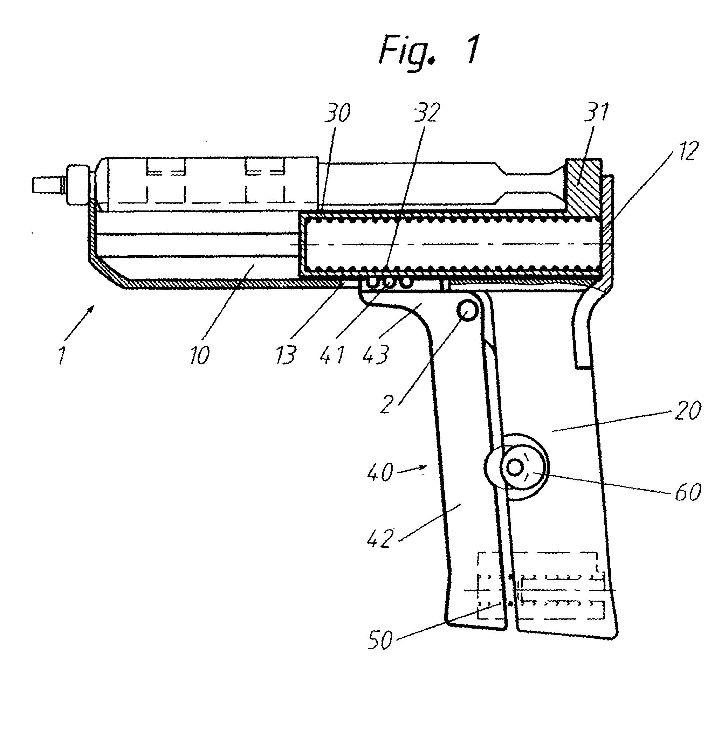

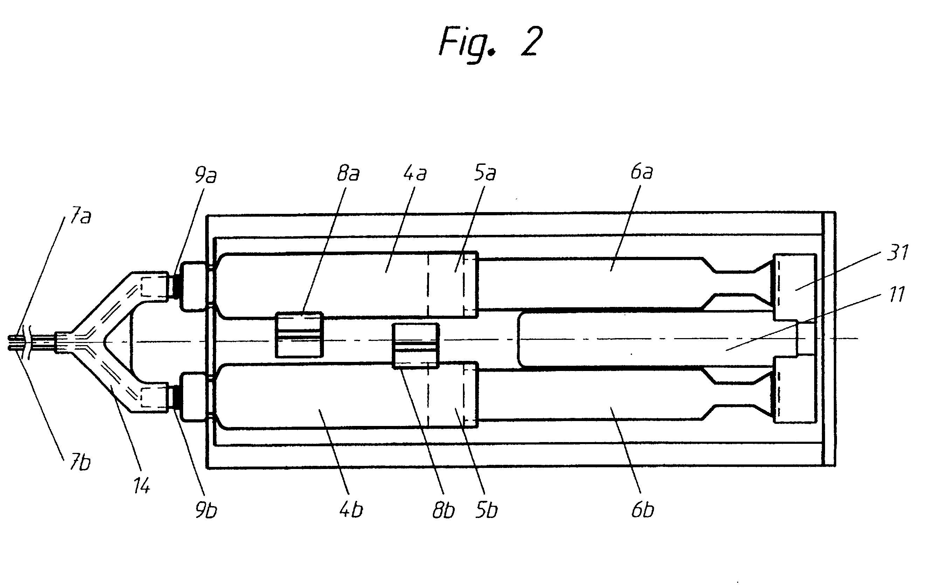

Wie in den Figuren 1 und 2 dargestellt, besteht das erfindungsgemässe Abgabegerät 1 aus einem pistolenförmigen Gehäuse, bestehend aus zwei Hauptelementen: einem ausgehöhlten flächigen Träger 10 und einem Haltegriff 20. Im Übergangsbereich zwischen dem Haltegriff 20 und dem flächigen Träger 10 ist eine Achse 2 angeordnet, welche einen Bremshebel 40 trägt. Der Anwender bedient das Abgabegerät 1 wie eine Pistole.

Auf dem flächigen Träger 10 werden parallel zwei Spritzampullen 4a,4b angeordnet. Die beiden Spritzampullen werden dabei von zwei Halterungen 8a,8b auf der Oberfläche des flächigen Trägers 10 derart gehalten, dass keine Bewegungen möglich sind. In den Spritzampullen 4a,4b befinden sich die beiden zu durchmischenden Flüssigkeiten. Spritzampullen sind im wesentlichen hohlzylindrische Behälter 4a,4b, welche distal mit einem beweglichen Kolben 5a,5b und auf der proximalen Seite durch ein Auslassstück 9a,9b abgeschlossen sind. Jeder Kolben 5a,5b ist zur Verlängerung mit einer Kolbenstange 6a,6b ausgestattet. Auf jede Auslassöffnung 9a,9b wird eine Kanüle 7a,7b derart aufgesetzt, dass eine Vermischung der beiden Flüssigkeiten erst auf der Oberfläche stattfindet, auf welche die beiden Flüssigkeiten aufgetragen werden. Die beiden Kanülen 7a,7b können dabei in einem tragenden Gehäuse 14 gehalten werden. Anstelle eines die Kanülen 7a,7b tragenden Gehäuses 14, kann auch eine Spritzvorrichtung verwendet werden, welche die beiden zu durchmischenden Flüssigkeiten auf eine Fläche aufspritzt.

Im inneren des holen, flächigen Trägers 10 befindet sich ein gleitbeweglicher Schlitten 30, auf den eine im distalen Bereich des Trägers 10 angeordnete Feder 32 derart wirkt, dass der Schlitten 30 in proximaler Richtung verschoben würde, wenn nicht eine Bremsfläche 41 dies verhindern würde. Die Feder 32 stösst dabei distal an die Rückwand 12 des flächigen Trägers 10 und proximal an den Schlitten 30.

Der flächige Träger 10 ist mit einer Öffnung 13 versehen, durch welche die Bremsfläche 41, auf den Schlitten 30 wirkt. Der Bremsmechanismus umfasst den an der Achse 2 angeordneten Bremshebel 40 und die Bremsfläche 41. Der Bremshebel 40 besteht aus einem längeren Teilstück 42 und einem dazu abgewinkelt angeordneten kürzeren Teilstück 43. Im Winkel zwischen dem kürzeren Teilstück 43 und dem längeren Teilstück 42 des Bremshebels 40 ist die Achse 2 angeordnet. Auf das längere Teilstück 42 des Bremshebels 40 wirkt eine Feder 50 derart, dass das längere Teilstück 42 vom Haltegriff 20 weggestossen wird, so dass die auf dem kürzeren Teilstück 43 angeordnete Bremsfläche 41 durch die Hebelwirkung gegen den Schlitten 30 gepresst wird, wodurch eine Bewegung des Schlitten 30 in proximaler Richtung verhindert wird.

Der Schlitten 30 ist derart ausgestaltet, dass eine T-förmige Platte 31, welche am distalen Ende des Schlittens 30 angeordnet ist, durch eine Längsöffnung 11 im flächigen Träger 10 herausragt und die beiden Kolbenstangen 6a,6b an ihrem distalen Ende berührt.

Wird das längere Teilstück 42 des Bremshebels 40 gegen den Druck der Feder 50 zum Haltegriff 20 gezogen, vermindert sich die Bremswirkung der Bremsfläche 41 auf den Schlitten 30, so dass dieser durch den Druck der Feder 32 in proximaler Richtung geschoben wird. Dabei werden die am T-Stück 31 anschlagenden Kolbenstangen 6a,6b und die damit verbundenen Kolben 5a,5b in proximaler Richtung geschoben. Diese Bewegung der Kolben 5a,5b in den Spritzampullen 4a,4b führt zu einer Verdrängung der Flüssigkeit durch die beiden Auslassöffnungen 9a,9b und die daran angeordneten Kanülen 7a,7b.

um eine ungewollte Betätigung des Bremshebels zu verhindern, wird zwischen dem längeren Teilstück 42 des Bremshebels 40 und dem Haltegriff 20 ein Sicherungsmechanismus 60 angebracht, welcher in geschlossener Position, keine Bewegung des Bremshebels 40 in Richtung Haltegriff 20 zulässt.

## Patentansprüche

1. Abgabegerät (1) zum gleichzeitigen Abgeben von Flüssigkeiten aus zwei mit Kolben (5a,5b) ausgerüsteten Flüssigkeitsbehältern (4a,4b), mit einer Betätigungseinrichtung, die zwei Abtriebsglieder (6a,6b) und ein die Abtriebsglieder bewegbares Schubelement (30) umfasst, und mit einem ersten Kraftelement (32), das auf das Schubelement (30) in Richtung Flüssigkeitsbehälter (4a,4b) wirkt, **dadurch gekennzeichnet, dass** durch ein auf das Schubelement (30) wirkendes Bremselement (41,50) die Wirkung des ersten Kraftelementes (32) aufgehoben werden kann, wobei durch manuelle Betätigung die Wirkung des Bremselementes (50,41) verändert werden kann.

2. Abgabegerät nach Anspruch 1, **dadurch gekennzeichnet, dass** das erste Kraftelement eine Feder (32) ist.

3. Abgabegerät nach Anspruch 1 oder 2, **dadurch gekennzeichnet, dass** das Bremselement (41,50) eine auf eine Bremsfläche (41) wirkende Feder (50) ist.

4. Abgabegerät nach einem der Ansprüche 1 bis 3, **dadurch gekennzeichnet, dass** die Flüssigkeitsbehälter (4a,4b) Spritzampullen sind.

5. Abgabegerät nach Anspruch 4, **dadurch gekennzeichnet, dass** die Spritzampullen (4a,4b) parallel angeordnet sind.

6. Abgabegerät nach einem der Ansprüche 1 bis 5, **dadurch gekennzeichnet, dass** das Abgabegerät (1) pistolenförmig ist.

7. Abgabegerät nach einem der Ansprüche 1 bis 6, **dadurch gekennzeichnet, dass** Abgabegerät (1) einen ausgehöhlten flächigen Träger (10) und einen Haltegriff (20) aufweist.

8. Abgabegerät gemäss Anspruch 7, **dadurch gekennzeichnet, dass** im Übergangsbereich zwischen dem Haltegriff (20) und dem flächigen Träger (10) eine Achse (2) angeordnet ist, welche einen Bremshebel (40) trägt

9. Abgabegerät nach Anspruch 8, **dadurch gekennzeichnet, dass** der Bremshebel (40) aus einem längeren Teilstück (42) und einem dazu abgewinkelt angeordneten kürzeren Teilstück (43), welches die Bremsfläche (41) trägt, besteht.

10. Abgabegerät nach einem der Ansprüche 1 bis 9, **dadurch gekennzeichnet, dass** ein Sicherungsmechanismus (60) eine ungewollte Betätigung des Bremshebels (40) verhindert.

## Claims

1. A delivery device (1) for the simultaneous delivery of fluids from two fluid containers (4a, 4b) provided with plungers (5a, 5b), comprising an actuating arrangement which includes two driven members (6a, 6b) and a thrust element (30) capable of moving the driven members, and a first force element (32) which acts on the thrust element (30) in the direction of the fluid containers (4a, 4b), **characterised in that** the action of the first force element (32) can be neutralised by a braking element (41, 50) acting on the thrust element (30), wherein the action of the braking element (50, 41) can be altered by manual actuation.

2. A delivery device according to claim 1 **characterised in that** the first force element is a spring (32).

3. A delivery device according to claim 1 or claim 2 **characterised in that** the braking element (41, 50) is a spring (50) acting on a braking surface (41).

4. A delivery device according to one of claims 1 to 3 **characterised in that** the fluid containers (4a, 4b) are injection ampoules.

5. A delivery device according to claim 4 **characterised in that** the injection ampoules (4a, 4b) are arranged parallel.

6. A delivery device according to one of claims 1 to 5 **characterised in that** the delivery device (1) is pistol-shaped.

7. A delivery device according to one of claims 1 to 6 **characterised in that** the delivery device (1) has a hollowed-out flat carrier (10) and a holding grip (20).

8. A delivery device according to claim 7 **characterised in that** arranged in the transitional region between the holding grip (20) and the flat carrier (10) is a spindle (2) which carries a braking lever (40).

9. A delivery device according to claim 8 **characterised in that** the braking lever (40) comprises a longer portion (42) and a shorter portion (43) which is arranged in angled relationship therewith and which carries the braking surface (41).

10. A delivery device according to one of claims 1 to 9 **characterised in that** a safety mechanism (60) prevents unintentional actuation of the braking lever (40).

## Revendications

1. Appareil de délivrance (1) pour délivrer simultanément des liquides à partir de deux récipients de liquides (4a, 4b) munis de pistons (5a, 5b), avec une installation d'actionnement qui comprend deux organes d'expulsion (6a, 6b) et un élément de poussée (30) qui met en mouvement les organes d'expulsion et avec un premier élément mécanique (32) qui agit sur l'élément de poussée (30) en direction des récipients de liquides (4a, 4b), **caractérisé en ce que** l'effet du premier élément mécanique (32) peut être supprimé par un élément de freinage (41, 50) qui agit sur l'élément de poussée (30), l'effet de l'élément de freinage (50, 41) pouvant être modifié par un actionnement manuel.

2. Appareil de délivrance selon la revendication 1 **caractérisé en ce que** le premier élément mécanique est un ressort (32).

3. Appareil de délivrance selon la revendication 1 ou 2 **caractérisé en ce que** l'élément de freinage (41, 50) est un ressort (50) qui agit sur une surface de freinage (41).

4. Appareil de délivrance selon l'une des revendications 1 à 3 **caractérisé en ce que** les récipients de liquides (4a, 4b) sont des seringues à usage unique.

5. Appareil de délivrance selon la revendication 4 **caractérisé en ce que** les seringues à usage unique (4a, 4b) sont disposées parallèlement.

6. Appareil de délivrance selon l'une des revendications 1 à 5 **caractérisé en ce que** l'appareil de délivrance (1) a une forme de pistolet.

7. Appareil de délivrance selon l'une des revendications 1 à 6 **caractérisé en ce que** l'appareil de délivrance (1) présente un support (10) plat évidé et une poignée (20).

8. Appareil de délivrance selon la revendication 7 **caractérisé en ce qu'**un axe (2) qui porte un levier de freinage (40) est placé dans la zone de transition entre la poignée (20) et le support (10) plat.

9. Appareil de délivrance selon la revendication 8 **caractérisé en ce que** le levier de freinage (40) est constitué d'un tronçon (42) assez long et d'un tronçon (43) plus court qui est disposé de façon recourbée par rapport à celui-ci et qui porte la surface de freinage (41).

10. Appareil de délivrance selon l'une des revendications 1 à 9 **caractérisé en ce qu'**un mécanisme de sécurité (60) empêche un actionnement involontaire du levier de freinage (40).
